# EUROPEAN PATENT SPECIFICATION

(11) **EP 1 793 796 B1**
(45) Date of publication and mention of the grant of the patent: **09.12.2009**
(21) Application number: 05786988.5
(22) Date of filing: 20.09.2005
(51) Int. Cl.: A61K 8/37, A61Q 11/00, A61K 8/97, A01N 31/16, A61K 8/34

(54) **USE OF AROMA SUBSTANCE MIXTURES AS AGENTS AGAINST BAD BREATH**
VERWENDUNG VON AROMASUBSTANZMISCHUNGEN ALS MITTEL GEGEN SCHLECHTEN ATEM
UTILISATION DE MÉLANGES DE SUBSTANCES AROMATIQUES COMME AGENTS CONTRE LA MAUVAISE HALEINE

(30) Priority: 24.09.2004 US 613023 P
(43) Date of publication of application: 13.06.2007
(73) Proprietor: Symrise GmbH & Co. KG, 37603 Holzminden (DE)
(72) Inventor: RABENHORST, Jürgen, 37671 Höxter (DE); MACHINEK, Arnold, 37603 Holzminden (DE); SONNENBERG, Steffen, 04416 Markkleeberg (DE); REINDERS, Gerald, 37671 Höxter (DE)
(74) Representative: Eisenführ, Speiser & Partner
(86) International application number: PCT/EP2005/054707
(87) International publication number: WO 2006/032668

(56) References cited:
- EP-A- 0 522 965
- WO-A-03/105794
- GB-A- 1 530 465
- US-A- 4 216 200
- US-B1- 6 231 836

## Description

The invention relates to mixtures comprising eugenol acetate and isoeugenol methyl ether and/or beta-homocyclocitral, the use thereof as agents against bad breath and corresponding methods. The invention furthermore relates to oral hygiene products containing these mixtures.

The healthy human mucous membrane of the oral and pharyngeal cavity and the solid tooth substance are colonized by a large number of non-pathogenic microorganisms. This so-called microflora of the oral cavity is not only harmless, it represents important protection for combating opportunistic or pathogenic germs.

One considerable problem of oral hygiene is offensive breath, also called bad breath, foetor ex oris or halitosis. This odour is formed by decomposition of residues of food and dead cells of the mucous membrane by microorganisms. The attack by Gram-positive and Gram-negative bacteria causes the bad breath. Anaerobic Gram-negative bacteria are mentioned above all as causative organisms in the literature (e.g. Bad Breath - A Multidisciplinary Approach. eds: D. van Steenberghe, M. Rosenberg, Leuven University Press, Leuven 1996; 111-121). Since social contacts are often impeded by bad breath, those affected have a great interest in remedying or preventing it.

Bad breath is to be differentiated here from pathological ulcerative stomatitis (stomatitis ulcerosa). While bad breath is a normal characteristic of human breath and therefore a merely cosmetic problem, ulcerative stomatitis is a disease. It is accompanied by a characteristic, very unpleasantly smelling note in the breath.

The object of the present invention was to discover active substances and agents against bad breath or against the microorganisms involved in the formation thereof.

The present invention primarily relates to a mixture comprising (a) eugenol acetate and isoeugenol methyl ether or (B) eugenol acetate and beta-homocyclocitral or (c) eugenol acetate, isoeugenol methyl ether and beta-homocyclocitral.

The present invention also relates to the use of a mixture according to the invention as an agent for inhibiting and/or preventing the growth of and/or for killing microorganisms which cause bad breath.

The present invention furthermore relates to a method of inhibiting and/or preventing the growth of and/or for killing microorganisms which cause bad breath with the following step:
- bringing microorganisms which cause bad breath into contact with a mixture according to the invention in an amount which is antimicrobially active against these microorganisms.

The invention also relates to a method of combating bad breath with the following step:
- introduction of a mixture according to the invention in an amount which is antimicrobially active against microorganisms which cause bad breath into the oral cavity and/or the pharyngeal cavity.

The invention also relates to an oral hygiene product containing a mixture according to the invention in an amount of at least 0.01 wt.%, based on the total weight of the oral hygiene product.

Further advantageous embodiments of the invention emerge from the patent claims, the following description and the examples.

Eugenol acetate (4-allyl-2-methoxyphenyl acetate; CAS No. 93-28-7) is known perse and can be represented by the following formula:

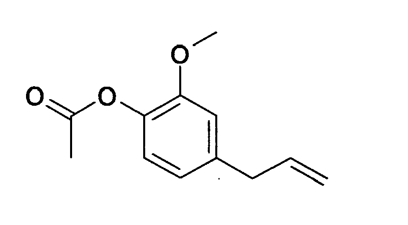

The smell of eugenol acetate can be described as clove-like, sweet and balsamic-fruity.

Clove oils are obtained from the flower buds, stem or leaves of the clove plant, each of these containing different amounts of the main components eugenol, eugenol acetate and caryophyllene.

The main constituent of clove oils is eugenol, which is responsible for the smell thereof and above all for the antimicrobial, antiseptic and analgesic action of clove oils. Eugenol has an antimicrobial activity against a large number of Gram-positive and Gram-negative bacteria.

The clove oils with the highest content of eugenol acetate are the oils which are obtained from the flower buds and which typically contain 75-90 wt.% eugenol, 4-15 wt.% eugenol acetate and 5-12 wt.% beta-caryophyllene. The weight ratio of eugenol acetate to eugenol in clove oils here is less than 1 : 5

Clove oils are in some cases employed in undiluted form in dental medicine.

In the field of oral care compositions, dilute clove oils, conventionally 1-2 % strength solutions, can be used as a mouth wash.

Mixtures and oral hygiene products which contain the eugenol acetate not in the form, i.e. not as a constituent, of a clove oil are preferred in the context of the present invention.

Isoeugenol methyl ether (methylisoeugenol, 1,2-dimethoxy-4-propenylbenzene, CAS No. 93-16-3) is known per se and can be represented by the following formulae: 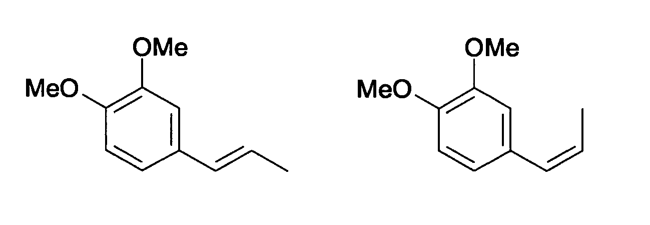 cis-Isoeugenol methyl ether, trans-isoeugenol methyl ether or any desired mixture of cis and trans isomers can be employed in the context of the present invention. Mixtures of 70 - 98 wt.% trans-isoeugenol methyl ether and 30 - 2 wt.% cis-isoeugenol methyl ether were employed in particular in our own studies.

The smell of isoeugenol methyl ether can be described as spicy and mildly clove-like.

Various essential oils contain isoeugenol methyl ether in small amounts. Depending on its origin, citronella grass oil (Cymbopogon nardus, citronella oil) contain, in addition to the main constituents citronellal, citronellol and geraniol, *inter alia* geranyl acetate and many other components. A distinction is made between two types of citronella grass oil, the Java type and the Sri Lanka (Ceylon) type, the main areas of cultivation of the Java type including not only Indonesia but also China and Taiwan. The oil which is predominantly used and is more valuable in perfume terms is of the Java type and comprises no isoeugenol methyl ether. The cintronella grass oil from Sri Lanka, which is less valuable in terms of smell, differs from the Java type by a content of isoeugenol methyl ether which is regularly in the range of 7 - 11 wt.% (DIN/ISO 3849-2000).

WO 99/18940 (corresponds to US 6,197,288) relates to a method and oral hygiene formulations for combating bad breath by means of mixtures of individual compounds from the range of odoriferous and aroma substances which mask malodour. This is not an antimicrobial action principle, but a masking / covering of the malodorous smell. Masking substances which are mentioned are, for example, geraniol, geranyl acetate, L-citronellol, eugenol and many other individual compounds. In a preferred embodiment, an aroma substance mixture which combats bad breath is said to comprise about 0.1 wt.% citronella grass oil, no regional origin being mentioned. It is thus to be assumed that it contained no isoeugenol methyl ether. According to WO 99/18940, an aroma substance mixture containing citronella grass oil is employed in an amount of only 0.03 to 0.1 wt.%.

US 3,947,570 discloses a mouth wash formulation which comprises 0 to 25 % alcohol and about 1 to 2 wt.%, based on the amount of alcohol employed in the formulation, of an aroma which acts as a denaturant. Citronella grass oil is also mentioned, in addition to a large number of other aromas, the regional origin not being mentioned. It is thus to be assumed that it contained no isoeugenol methyl ether.

Mixtures and oral hygiene products which contain isoeugenol methyl ether not in the form, i.e. not as a constituent, of a citronella grass oil are preferred in the context of the present invention.

DE 19612340 (corresponds to US 6,514,551) describes a process for improving the life of and/or stabilizing microbially perishable products by means of a process auxiliary which comprises at least one microbicidally acting aroma substance, eugenol acetate and also the compounds eugenol, isoeugenol and methyleugenol, which are structurally related to isoeugenol methyl ether, being mentioned among a large number of aroma substances. This process auxiliary can be employed, for example, in the form of lubricants, emulsifying and cleaning compositions and cutting or release agents. In this procedure, only the surfaces or cut areas of the foodstuffs are charged with the process auxiliaries. The activity against various microorganisms, in particular organisms which cause decay (e.g. A. niger, Enterobacter, Lactobacilli) but which play no role in the formation of bad breath, has been investigated.

beta-Homocyclocitral (2,6,6-trimethyl-1-cyclohexen-1-acetaldehyde; CAS No. 472-66-2, FEMA No. 3474) is known per se and can be represented by the following formula:

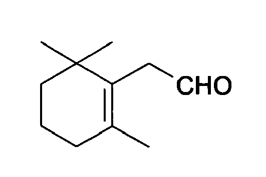

The smell is usually defined as camphorous, woody, oily, fruity and also herbal.

Extracts and distillates obtainable from the leaves of sugar-cane (Saccharum officinarum) and some aroma substances contained therein are described in EP 0 941 671. beta-Homocyclocitral was found as a trace component in an extract and a distillate. EP 0 941 671 furthermore describes the intensification of the sweetness and the (complete) reduction of the bitterness of an edible formulation, for example a diet fizzy drink, by means of beta-homocyclocitral in concentrations in the range of 1 to 20 ppb.

Mixtures and oral hygiene products which contain beta-homocyclocitral not in the form, i.e. not as a constituent, of an extract or distillate from leaves of sugar-cane, such as is described e.g. in EP 0 941 671, are preferred in the context of the present invention.

In the present text, oral hygiene products are understood as meaning the formulations familiar to the person skilled in the art for cleansing and care of the oral cavity and the pharyngeal cavity and for refreshing the breath. Known and customary oral hygiene formulations are both creams, gels, pastes, foams, emulsions, suspensions, aerosols and sprays, and capsules, granules, pastilles, tablets, bonbons or chewing gums, without this list of presentation forms being limiting in respect of the possible uses. Such formulations serve to cleanse and care for the tooth substance and oral cavity and to refresh the breath.

A mixture according to the invention can be incorporated largely universally into the most diverse presentation forms of oral hygiene products without having to be limited to one or a few specific presentation forms, i.e. a mixture according to the invention harmonizes with a very large number of conventional cosmetic auxiliary substances and additives.

It has furthermore emerged that a mixture according to the invention or an oral hygiene product according to the invention can both effectively reduce or eliminate bad breath and prevent formation thereof, i.e. is also capable of acting preventively.

A mixture according to the invention or an oral hygiene product according to the invention effectively combats bad breath without noticeably harming the physiological flora of the oral and pharyngeal cavity.

It has been found that a mixture according to the invention or an oral hygiene product according to the invention can completely or partly prevent the growth of Gram-positive and Gram-negative bacteria of the oral and pharyngeal cavity which are responsible for bad breath.

It has been found that a mixture according to the invention or an oral hygiene product according to the invention can completely or partly prevent the formation of components which cause bad breath.

In particular, a mixture according to the invention or an oral hygiene product according to the invention is capable of inhibiting and/or preventing the growth of microorganisms which cause bad breath, and/or of killing these, which are chosen from the group consisting of: Eubacterium, Fusobacterium, Haemophilus, Neisseria, Porphyromonas, Prevotella, Treponema and Veillonella species, in particular Fusobacterium nucleatum, Porphyromonas endodontalis, Porphyromonas gingivalis, Prevotella intermedia, Prevotella loeschii and Treponema denticola.

A mixture according to the invention or an oral hygiene product according to the invention is distinguished in particular by a very good action against anaerobic and microaerophilic bacteria.

It was furthermore astonishing that a mixture according to the invention or an oral hygiene product according to the invention has a particularly good action against the particularly pronounced morning bad breath which is typically to be perceived in the morning after getting up.

In addition, it has emerged that a mixture according to the invention can completely or partly prevent the decay of oral hygiene products due to attack by Gram-positive and/or Gram-negative bacteria when it is added to these products in an active amount. A mixture according to the invention can thus also simultaneously act as a preservative here.

If a mixture according to the invention comprises eugenol acetate and isoeugenol methyl ether in accordance with alternatives (a) and (c), a weight ratio of eugenol acetate to isoeugenol methyl ether in the range of from 10 : 1 to 1 : 10 is advantageous, and a weight ratio in the range of from 5 : 1 to 1 : 5, is preferred, particularly preferably in the range of from 4 : 1 to 1 : 3 and very particularly preferably in the range of from 3 : 1 to 1 : 2.

If a mixture according to the invention comprises eugenol acetate and beta-homocyclocitral in accordance with alternatives (b) and (c), a weight ratio of eugenol acetate to beta-homocyclocitral in the range of from 100 : 1 to 1 : 5 is advantageous, and a weight ratio in the range of from 75 : 1 to 1 : 1, is preferred, particularly preferably in the range of from 75 : 1 to 10 : 1 and very particularly preferably in the range of from 60 : 1 to 20 : 1.

In a preferred embodiment according to alternative (c), a mixture according to the invention comprises eugenol acetate, isoeugenol methyl ether and beta-homocyclocitral in the abovementioned preferred weight ratios.

The total content of the constituents eugenol acetate, isoeugenol methyl ether and beta-homocyclocitral here is advantageously in the range of from 10 to 100 wt.%, preferably in the range of from 20 to 100 wt.%, particularly preferably in the range of from 30 to 100 wt.% and very particularly preferably in the range of from 40 to 100 wt.%, based on the total weight of the mixture according to the invention.

In a preferred embodiment of alternative (a), a mixture according to the invention comprises eugenol acetate, isoeugenol methyl ether and one or more further substances chosen from the group consisting of farnesol, myristicin, apiole, parsley oil and thymol.

In a particularly preferred embodiment of alternative (c), a mixture according to the invention comprises eugenol acetate, isoeugenol methyl ether, beta-homocyclocitral and one or more further substances chosen from the group consisting of farnesol, myristicin, apiole, parsley oil and thymol.

Thymol and farnesol (in the example of the 2-trans-6-trans isomer) can be represented by the following structural formulae:

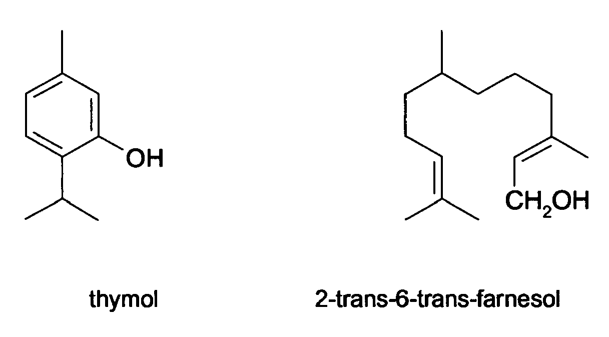

Myristicin [1-allyl-3-methoxy-4,5-(methylenedioxy)-benzene] and apiole [1-allyl-2,5-dimethoxy-3,4-(methylenedioxy)-benzene] can be represented by the following structural formulae:

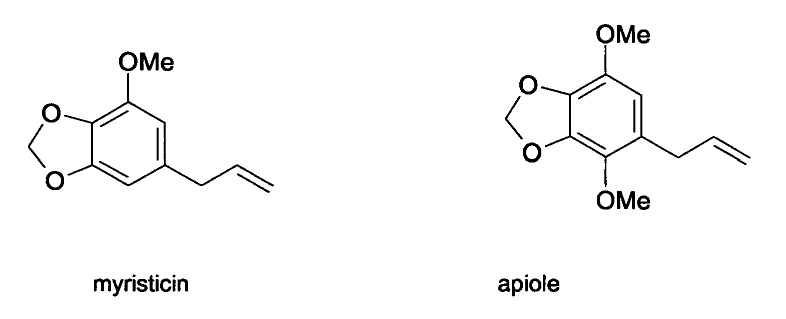

The total content of the constituents eugenol acetate, isoeugenol methyl ether, beta-homocyclocitral and the further substance(s) chosen from the group consisting of farnesol, myristicin, apiole, parsley oil and thymol here is advantageously in the range of from 15 to 100 wt.%, preferably in the range of from 25 to 100 wt.%, particularly preferably in the range of from 35 to 100 wt.% and very particularly preferably in the range of from 45 to 100 wt.%, based on the total weight of the particularly preferred mixture according to the invention.

Parsley oil is preferably employed in a mixture according to the invention in the form of a parsley seed oil, which naturally contains myristicin and apiole.

Thymol can be employed in a mixture according to the invention in the pure form or in the form, i.e. as a constituent, of a thymol-containing natural oil, such as, for example, a thyme or oregano oil.

All four possible double bond isomers of farnesol, as any desired mixture or as an individual isomer, can in principle be employed, and 2-trans-6-trans-farnesol and 2-cis-6-trans-farnesol or a mixture thereof are preferred. Farnesol can also be employed in a mixture according to the invention in the form, i.e. as a constituent, of a famesol-containing natural oil.

Preferred mixtures (E-1 to E-6) according to the invention are summarized in Table 1. The amounts stated relate to parts by weight.

**Table 1:**

| Substance | E-1 | E-2 | E-3 | E-4 | E-5 | E-6 |
|---|---|---|---|---|---|---|
| Eugenol acetate | 50 | 98 | 40 | 50 | 45 | 50 |
| Isoeugenol methyl ether | 50 | - | 51 | 30 | 45 | 34 |
| beta-Homocyclocitral | - | 2 | 1 | 1 | 0.5 | 1 |
| Farnesol | - | - | 3 | 10 | 1 | 5 |
| Parsley seed oil | - | - | 5 | 5 | 7.5 | 5 |
| Thymol | - | - | - | 4 | 1 | 5 |

An oral hygiene product having a content of a mixture according to the invention in the range of from 0.01 (100 ppm) to 10 wt.%, in particular having a content of from 0.025 (250 ppm) to 5 wt.%, preferably having a content of from 0.05 (500 ppm) to 5 wt.% and particularly preferably 0.1 to 3 wt.%, based on the total weight of the product, is preferred according to the invention.

In a very particularly preferred embodiment, the content of a mixture according to the invention is in the range of from 0.16 to 2.75 wt.%, based on the total weight of the product.

An oral hygiene product having a content of eugenol acetate of from 0.005 (50 ppm) to 5 wt.%, in particular having a content of from 0.01 (100 ppm) to 3 wt.%, preferably having a content of from 0.025 (250 ppm) to 3 wt.% and particularly preferably 0.05 (500 ppm) to 2 wt.%, based on the total weight of the product, is preferred according to the invention.

In a very particularly preferred embodiment, the content of eugenol acetate is in the range of from 0.1 to 1.5 wt.%, based on the total weight of the product.

An oral hygiene product having a content of isoeugenol methyl ether of from 0.005 (50 ppm) to 5 wt.%, in particular having a content of from 0.01 (100 ppm) to 3 wt.%, preferably having a content of from 0.025 (250 ppm) to 2 wt.%, and particularly preferably 0.06 (600 ppm) to 3 wt.%, based on the total weight of the product, is preferred according to the invention.

In a very particularly preferred embodiment, the content of isoeugenol methyl ether is in the range of from 0.06 (600 ppm) to 1 wt.%, based on the total weight of the product.

In the most preferred embodiment, the content of eugenol acetate is in the range of from 0.1 to 1 wt.% and that of isoeugenol methyl ether is in the range of from 0.08 (800 ppm) to 1 wt.%, based on the total weight of the product.

According to the invention, beta-homocyclocitral is preferably employed in oral hygiene products with a content of from 0.001 (10 ppm) to 1.0 wt.%, in particular with a content of from 0.002 (20 ppm) to 0.5 wt.%, particularly preferably with a content of from 0.004 (40 ppm) to 0.5 wt.% and very particularly preferably 0.005 (50 ppm) to 0.25 wt.%, based on the total weight of the product.

If a mixture according to the invention or an oral hygiene product according to the invention also contains eugenol, a weight ratio of eugenol acetate to eugenol of greater than 1 : 3 is advantageous, and a weight ratio of greater than 1 : 1 is preferred, particularly preferably greater than 2 : 1 and very particularly preferably greater than 10 : 1.

The taste of eugenols is very clove-like and phenolic and is often found to be unpleasant and medicinal, also in oral hygiene products.

A mixture according to the invention or an oral hygiene product according to the invention which contains only a very small amount of eugenol or no eugenol is preferred according to the invention. In a preferred embodiment, a mixture according to the invention or an oral hygiene product according to the invention is substantially free from eugenol. In the present case, substantially free from eugenol means that the mixture or the product contains eugenol at most in an amount which is not active against bad breath.

The taste of eugenol acetate (in oral hygiene products), especially at relatively high dosages, also is not always found to be pleasant, although this is significantly less clove-like and medicinal than that of eugenol.

Furthermore, the taste of isoeugenol methyl ether (in oral hygiene products), especially at relatively high dosages, also is not always found to be pleasant, although this is only weakly clove-like and medicinal.

Consequently, the taste of a mixture according to the invention (in oral hygiene products), especially at relatively high dosages, is not always found to be pleasant and can be described as noticeably clove-like and medicinal.

In the context of the present invention, there was thus the subsequent object of achieving a modification of the taste of a mixture according to the invention or of an oral hygiene product according to the invention such that this has an improved, pleasanter and less clove-like and/or medicinal taste.

It has been possible to achieve this subsequent object by a mixture according to the invention comprising menthol or an oral hygiene product according to the invention containing an aroma composition comprising menthol.

In this context, menthol not only imparts the menthol-specific cooling freshness, but at the same time reduces the clove-like and medicinal taste impression of the mixture according to the invention.

A further aspect of the present invention therefore relates to a mixture according to the invention comprising a sensorially active amount of menthol.

A further aspect of the present invention relates to an oral hygiene product containing 0.01 (100 ppm) to 10 wt.% of a mixture according to the invention and an aroma composition containing menthol.

An aroma composition in the context of the present invention comprises menthol and one or more further substances having a sensory action in each case in a sensorially active amount.

An oral hygiene product wherein the weight ratio of the sum of the mixture constituents eugenol acetate, isoeugenol methyl ether and beta-homocyclocitral to an aroma composition comprising menthol is in the range of from 50 : 1 to 1 : 8, preferably from 20 : 1 to 1 : 4 is prepared according to the invention.

The weight ratio of sum of the mixture constituents eugenol acetate, isoeugenol methyl ether and beta-homocyclocitral to menthol, i.e. the sum of all the menthol isomers, is advantageously in the range of 5 : 1 to 1 : 5, preferably in the range of 3 : 1 to 1 : 3. Of the menthol isomers, racemic menthol and 1-menthol are preferred.

Menthol can be employed here in the pure form (natural or synthetic) and/or as a constituent of natural oils and/or menthol-containing fractions of natural oils, in particular in the form of peppermint oils, such as Mentha arvensis or Mentha piperita or menthol-containing fractions thereof.

It is of course possible and advantageous to prepare a mixture according to the invention (with menthol) from a base mixture according to the invention (without menthol) and an aroma composition comprising menthol, to subsequently incorporate this into an oral hygiene formulation and to prepare an oral hygiene product according to the invention in this manner.

It has moreover been found that a mixture according to the invention or an oral hygiene product according to the invention has a taste which is improved further if it comprises an aroma composition which also contains, in addition to menthol, one or more substances having an aniseed note.

Advantageous substances having an aniseed note are: anethole, anisole, aniseed oil, star aniseed oil and fennel oil, and in addition also anisaldehyde and anisyl alcohol. Anethole and anethole-containing essential oils are preferred.

Substances having an aniseed note, in particular anethole, impart to a mixture according to the invention (having a content of menthol) or an oral hygiene product according to the invention (having a content of menthol) a certain sweetness and a gentler, softer taste profile, and at the same time reduce the clove-like and medicinal taste impression of the mixture according to the invention.

The weight ratio of sum of the mixture constituents eugenol acetate, isoeugenol methyl ether and beta-homocyclocitral to anethole is advantageously in the range of 20 : 1 to 1 : 5, preferably in the range of 10 : 1 to 1 : 2.

The anethole can be used here as cis- or trans-anethole or in the form of mixtures of the isomers. Anethole can be employed here in the pure form (natural or synthetic) and/or as a constituent of natural oils and/or anethole-containing fractions of natural oils, in particular in the form of aniseed oil, star aniseed oil or fennel oil or anethole-containing fractions thereof.

It has moreover been found that a mixture according to the invention or an oral hygiene product according to the invention has a taste which is improved further if it comprises an aroma composition which also contains, in addition to menthol and optionally one or more substances having an aniseed note, eucalyptol (1,8-cineol).

Eucalyptol can be employed here in the pure form (natural or synthetic) and/or as a constituent of natural oils and/or eucalyptol-containing fractions of natural oils, for example in the form of bay (leaf) oil, but eucalyptus oils from Eucalyptus fruticetorum and/or Eucalyptus globulus and/or eucalyptol-containing fractions thereof are preferred.

The weight ratio of sum of the mixture constituents eugenol acetate, isoeugenol methyl ether and beta-homocyclocitral to eucalyptol is advantageously in the range of 100 : 1 to 1 : 5, preferably in the range of 60 : 1 to 1 : 1.

Eucalyptol imparts to a mixture according to the invention (having a content of menthol) or an oral hygiene product according to the invention (having a content of menthol) a fresh head note and slightly camphorous aspects and at the same time reduces the clove-like and medicinal taste impression of a mixture according to the invention.

It has moreover been found that a mixture according to the invention or an oral hygiene product according to the invention has a taste which is improved further if it comprises an aroma composition which also contains, in addition to menthol and optionally one or more substances having an aniseed note and/or eucalyptol, at least one or more herbal components.

Suitable herbal components are, in particular, basil oil, camomile oil, nutmeg oil, (in particular nutmeg blossom oil = mace oil), myrrh oil, oregano oil, rosemary oil, sage oil (clary sage, Dalmatian or Spanish sage oil), thyme oil, juniper oil (in particular juniper berry oil) or fractions thereof.

In addition, it is advantageous if the oral hygiene product according to the invention contains at least one further aroma substance from the following group: methone, isomenthone, isopulegol, menthyl acetate, limonene, pinene (optionally as a constituent of a Eucalyptus globulus oil), carvone (optionally as a constituent of a spearmint oil) and methyl salicylate (optionally as a constituent of a wintergreen oil).

A preferred embodiment relates to a mixture according to the invention comprising:
5 to 95 wt.%, preferably 20 to 80 wt.%, particularly preferably 30 to 70 wt.% of the constituents eugenol acetate, isoeugenol methyl ether and beta-homocyclocitral
   and
5 to 95 wt.%, preferably 20 to 80 wt.%, particularly preferably 30 to 70 wt.% of the constituents menthol and optionally one or more substances having an aniseed note and/or eucalyptol and/or one or more herbal components.

Mixtures according to the invention which are furthermore preferred are those which comprise an aroma composition comprising racemic menthol and/or I-menthol, anethole, eucalyptol and optionally one or more herbal components, and these in turn preferably in the abovementioned preferred amounts and ratios.

In a preferred embodiment, an oral hygiene product according to the invention contains:
0.025 to 5 wt.% of the constituents eugenol acetate, isoeugenol methyl ether and beta-homocyclocitral,
0.025 to 10 wt.%, preferably 0.025 to 5 wt.% of an aroma composition which contains menthol and optionally one or more substances having an aniseed note and/or eucalyptol and/or one or more herbal components. The weight ratio of the sum of the said constituents to menthol here is preferably in the range of 5 : 1 to 1 : 5.

In this context, the weight data for the said constituents of the aroma composition in each case relate to the total weight of the finished oral hygiene product.

In a further preferred embodiment, an oral hygiene product according to the invention contains:
0.1 to 3 wt.% of the constituents eugenol acetate, isoeugenol methyl ether and beta-homocyclocitral and
0.05 to 5 wt.%, preferably 0.1 to 3 wt.% of an aroma composition which contains racemic menthol and/or I-menthol, anethole, eucalyptol and optionally one or more herbal components, the weight ratio of the sum of the said constituents to menthol being in the range of 15 : 1 to 1 : 5, preferably in the range of 3 : 1 to 1 : 3.

In this context, the weight data for the said constituents and the aroma composition in each case relate to the total weight of the finished oral hygiene product.

In the preferred embodiments of mixtures according to the invention or of oral hygiene products according to the invention, the aroma composition in turn preferably comprises:
5 to 80 wt.%, preferably 10 to 70 wt.% menthol;
1 to 25 wt.%, preferably 2 to 20 wt.% of one or more substances having an aniseed note, preferably anethole;
0.5 to 50 wt.%, preferably 1 to 30 wt.% eucalyptol;
   and optionally
0.01 to 25 wt.%, preferably 0.1 to 15 wt.% of one or more herbal components, in each case based on the total weight of the aroma composition.

The sum of menthol, substance(s) having an aniseed note, eucalyptol and optionally herbal component(s) in the aroma composition here is preferably greater than or equal to 40 wt.%, particularly preferably greater than or equal to 50 wt.%, and particularly preferably greater than or equal to 60 wt.%, based on the total weight of the aroma composition.

The mixtures comprising menthol or oral hygiene products containing an aroma composition comprising menthol which are preferred according to the invention not only prevent or combat bad breath, but at the same time impart a fresh, pleasant taste and breath.

It is advantageous to buffer the oral hygiene products according to the invention. A pH range of from 3.5 to 10.0 is advantageous.

A mixture according to the invention can be incorporated without problems into the usual oral hygiene formulations for oral hygiene products. Preferred oral hygiene products are, for example, dental creams, toothpastes, dental gels, mouth washes, mouth rinses, liquids for gargling and mouth or throat sprays, as well as sucking pastilles, sucking tablets, bonbons, chewing gums, chewing bonbons and dental care chewing gums.

It is also possible and usually advantageous to combine a mixture according to the invention with other starting substances, for example with other antimicrobially active substances, aroma substances, flavouring substances and/or auxiliary substances.

The oral hygiene products according to the invention can contain auxiliary substances such as are conventionally used in such formulations, e.g. preservatives, abrasives, antibacterial agents, antiinflammatory agents, irritation-preventing agents, irritation-inhibiting agents, antimicrobial agents, antioxidants, astringents, antiseptic agents, antistatics, binders, buffers, carrier materials, chelating agents, cell stimulants, cleansing agents, care agents, surface-active substances, deodorizing agents, softeners, bactericides, emulsifiers, enzymes, essential oils, film-forming agents, fixatives, foam-forming agents, foam stabilizers, substances for preventing foaming, foam boosters, gelling agents, gel-forming agents, moisture-donating agents, moisturizing substances, moisture-retaining substances, bleaching agents, optically brightening agents, dirt-repellent agents, friction- reducing agents, lubricants, opacifying agents, covering agents, gloss agents, polymers, powders, proteins, abrading agents, silicones, skin soothing agents, skin cleansing agents, skin care agents, skin healing agents, cooling agents, skin cooling agents, warming agents, skin warming agents, stabilizers, suspending agents, thickeners, vitamins, oils, waxes, fats, phospholipids, saturated fatty acids, mono- or polyunsaturated fatty acids, α-hydroxy acids, polyhydroxy-fatty acids, liquefiers, dyestuffs, colour-protecting agents, pigments, aromas, flavouring substances, odoriferous substances or other conventional constituents of a cosmetic or oral hygiene formulation, such as alcohols, polyols, electrolytes, organic solvents, sweeteners, sugar substitutes, silicas, calcium carbonate, calcium hydrogen phosphate, aluminium oxide, fluorides, salts of zinc, tin, potassium, sodium and strontium, pyrophosphates, hydrogen peroxide and hydroxyapatite.

If the oral hygiene product is a solution or lotion, solvents which can be used are, for example: water or aqueous solutions, oils, such as triglycerides of capric or of caprylic acid or also alcohols, diols or polyols of low C number and ethers thereof, preferably ethanol, isopropanol, propylene glycol, glycerol or ethylene glycol. Mixtures of the abovementioned solvents can of course also be used.

Examples of flavouring substances or aromas which can be a constituent of an oral hygiene product according to the invention, in addition to or as a constituent of a mixture according to the invention, are to be found e.g. in K. Bauer, D. Garbe, H. Surburg, Common Fragrance and Flavor Materials, 4th ed., Wiley-VCH, Weinheim 2001 or also in S. Arctander, Perfume and Flavor Chemicals, vol. I and II, Montclair, N. J., 1969, author and publisher.

Examples which may be mentioned of natural aromas which can be a constituent of an oral hygiene product according to the invention, in addition to or as a constituent of a mixture according to the invention, are: peppermint oils, spearmint oils, Mentha arvensis oils, aniseed oils, clove oils, citrus oils, camphor oils, cinnamon oils, cinnamon bark oils, wintergreen oils, eucalyptus oils, Eucalyptus citriodora oils, fennel oils, ginger oils, camomile oils, caraway oils, citronella oils, lime oils, orange oils, bergamot oils, grapefruit oils, mandarin oils, rose oils, geranium oils, sage oils, parsley seed oils, yarrow oils, star aniseed oils, basil oil, bitter almond oils, thyme oils, juniper berry oils, rosemary oils, angelica root oils, vanilla extracts, and fractions thereof or contents isolated therefrom.

Examples which may be mentioned of uniform aroma substances which can be a constituent of an oral hygiene product according to the invention, in addition to or as a constituent of a mixture according to the invention, are: anethole, menthol, menthone, isomenthone, menthyl acetate, menthyl propionate, menthofuran, mint lactone, eucalyptol (1,8-cineol), limonene, eugenol, thymol, pinene, sabinene hydrate, 3-octanol, carvone, gamma-octalactone, gamma-nonalactone, germacrene D, viridiflorol, 1,3E,5Z-undecatriene, isopulegol, piperitone, 2-butanone, ethyl formate, 3-octyl acetate, isoamyl isovalerate, hexanol, hexanal, cis-3-hexenol, linalool, alpha-terpineol, cis- and trans-carvyl acetate, p-cymol, damascenone, damascones, rose oxide, fenchol, acetaldehyde diethyl acetal, 1-ethoxyethyl acetate, cis-4-heptenal, isobutyraldehyde, isovaleraldehyde, cisjasmone, methyl dihydrojasmonate, anisaldehyde, methyl salicylate, 2'-hydroxypropiophenone, menthyl methyl ether, myrtenyl acetate, 2-phenylethyl alcohol, 2-phenylethyl isobutyrate, 2-phenylethyl isovalerate, cinnamaldehyde, geraniol and nerol. In the case of chiral compounds, the aroma substances can be in the form of a racemate or in the form of an individual enantiomer or in the form of an enantiomerically enriched mixture.

Advantageous aromas or aroma substances which can be a constituent of an oral hygiene product according to the invention, in addition to or as a constituent of a mixture according to the invention, are, for example, anisole, basil oil, bitter almond oil, camphor oil, citronella oil, citrus oils, Eucalyptus citriodora oil, eucalyptus oil, camomile oil, spearmint oil, lime oil, mandarin oil, clove oil, orange oil, peppermint oil, sage oil, thyme oil, wintergreen oil, cinnamon oil, cinnamon bark oil, I-menthol, menthone, 1,8-cineol (eucalyptol), carvone, alpha-terpineol, methyl salicylate, 2'-hydroxypropiophenone and menthyl methyl ether.

Compounds having a physiological cooling effect (cooling substances) which can be a constituent of an oral hygiene product according to the invention, in addition to or as a constituent of a mixture according to the invention, are, for example, I-menthol, menthone glycerol acetal, menthyl lactate, substituted menthyl-3-carboxylic acid amides (e.g. menthyl-3-carboxylic acid N-ethylamide), 2-isopropyl-N,2,3-trimethylbutanamide, substituted cyclohexanecarboxylic acid amides, 3-menthoxypropane-1,2-diol, 2-hydroxyethyl menthyl carbonate, 2-hydroxypropyl menthyl carbonate, N-acetylglycine menthyl ester, menthyl hydroxycarboxylic acid esters (e.g. menthyl 3-hydroxybutyrate), monomenthyl succinate, 2-mercaptocyclodecanone, menthyl 2-pyrrolidin-5-onecarboxylate, 2,3-dihydroxy-p-menthane, 3,3,5-trimethylcyclohexanone glycerol ketal, 3-menthyl 3,6-di- and tri-oxaalkanoates, 3-menthyl methoxyacetate, ilcilin and I-menthyl methyl ether. I-Menthol, menthone glycerol acetal, menthyl lactate, menthyl-3-carboxylic acid N-ethylamide, 3-menthoxypropane-1,2-diol, 2-hydroxyethyl menthyl carbonate, 2-hydroxypropyl menthyl carbonate, monomenthyl succinate, menthyl 2-pyrrolidin-5-onecarboxylate and I-menthyl methyl ether are preferred.

Oral hygiene products which contain at least one, particularly preferably at least two further cooling substances, in addition to I-menthol, are preferred according to the invention.

Components which cause a warming, sharp, tingling or prickling sensation on the skin or on the mucous membranes, in particular aroma substances having a heat-generating effect and/or sharp-tasting compounds (sharp substances), and which can be a constituent of an oral hygiene product according to the invention, in addition to or as a constituent of a mixture according to the invention, are, for example, capsaicin, dihydrocapsaicin, gingerol, paradol, shogaol, piperine, paprika powder, chilli pepper powder, extracts from paprika, extracts from pepper; extracts from chilli pepper; extracts from ginger roots, extracts from Aframomum melgueta, extracts from Spilanthes acmella, extracts from Kaempferia galanga, extracts from Alpinia galanga, carboxylic acid N-vanillylamides, in particular nonanoic acid N-vanillylamide, 2-nonenoic acid amides, in particular 2-nonenoic acid N-isobutylamide and 2-nonenoic acid N-4-hydroxy-3-methoxyphenylamide, alkyl ethers of 4-hydroxy-3-methoxybenzyl alcohol, in particular 4-hydroxy-3-methoxybenzyl n-butyl ether, alkyl ethers of 3-hydroxy-4-methoxybenzyl alcohol, alkyl ethers of 3,4-dimethoxybenzyl alcohol, alkyl ethers of 3-ethoxy-4-hydroxybenzyl alcohol, alkyl ethers of 3,4-methylenedioxybenzyl alcohol, (4-hydroxy-3-methoxyphenyl)acetic acid amides, in particular (4-hydroxy-3-methoxyphenyl)acetic acid N-n-octylamide, nicotinaldehyde, methyl nicotinate, propyl nicotinate, 2-butoxyethyl nicotinate, benzyl nicotinate and 1-acetoxychavicol.

Further components which can be a constituent of an oral hygiene product according to the invention, in addition to or as a constituent of a mixture according to the invention, are e.g. substances for improving oral hygiene, such as, for example, dental care and/or refreshing substances. The substances for improving oral hygiene include, for example, substances for combating or preventing plaque, tartar or caries, and those for combating or preventing bad breath. Refer ence may be made in this connection to US 5,043,154. Examples which may be mentioned are Zn salts, such as Zn citrate and Zn fluoride, Sn salts, such as Sn fluorides, Cu salts, fluorides, e.g. amine fluorides, alkali metal fluorides, such as Na fluoride, alkaline earth metal fluorides and ammonium fluoride, phosphates, pyrophosphates, fluorophosphates, such as Na monofluorophosphate and Al mono- and Al difluorophosphate, alpha-ionones, geraniol, thymol, isomenthyl acetate, panthenol (provitamin B₅), xylitol, allantoin, niacinamide (vitamin B₃), tocopheryl acetate (vitamin E acetate) and poloxamer.

An oral hygiene product according to the invention can also contain one or more further antimicrobial active compounds for improving oral hygiene, in addition to the antimicrobial constituents mentioned for a mixture according to the invention. These active compounds can be hydrophilic, amphoteric or hydrophobic in nature. Examples which may be mentioned are: Triclosan, chlorhexidine and salts, peroxides, phenols and salts thereof, domiphen bromide (phenododecinium bromide), bromochlorophene, Zn salts, chlorophylls, Cu salts, Cu gluconate, Cu-chlorophyll, sodium lauryl sulfate, quaternary monoammonium salts, such as coconut alkyl-benzyldimethylammonium chloride, or also pyridinium salts, such as cetylpyridinium chloride. In addition to individual active compounds, mixtures of active compounds or natural extracts or fractions thereof containing active compounds can be employed, such as e.g. those obtainable from neem, berberitze, fennel, green tea, marigold, camomile, rosemary, thyme, propolis or turmeric.

An oral hygiene product according to the invention can contain antioxidants, in addition to or as a constituent of a mixture according to the invention, and examples which may be mentioned here are: carotenoids, carotenes (e.g. α-carotene, β-carotene and lycopene) and derivatives thereof, flavonoids, quercetin (metal) chelators (e.g. α-hydroxy-fatty acids. fatty acids (palmic acids), phytic acid, lactoferrin, EDTA and EGTA), α-hydroxy acids (e.g. citric acid, lactic acid and malic acid), folic acid and derivatives thereof, ubiquinone and ubiquinol and derivatives thereof, vitamin C and derivatives (e.g. ascorbyl palmitate, Mg ascorbyl phosphate and ascorbyl acetate), tocopherols and derivatives (e.g. vitamin E acetate), vitamin A and derivatives (vitamin A palmitate), ferulic acid and derivatives thereof, butylhydroxytoluene (BHT), butylhydroxyanisole, zinc and derivatives thereof (e.g. ZnO and ZnSO₄), selenium and derivatives thereof (e.g. selenium-methionine); and constituents, extracts or fractions thereof isolated from plants, e.g. from tea, green tea, algae, grape seeds, wheat germ, camomile, rosemary and oregano.

An oral hygiene product according to the invention can contain dyestuffs, colouring agents or pigments, in addition to or as a constituent of a mixture according to the invention, and examples which may be mentioned here are: lactoflavin (riboflavin), beta-carotene, riboflavin 5'-phosphate, alpha-carotene, gamma-carotene, cantaxanthin, erythrosine, curcumin, quinoline yellow, Yellow Orange S, tartrazine yellow, bixin, norbixin (annatto, orlean), capsanthin, capsorubin lycopene, beta-apo-8'-carotenal, beta-apo-8'-carotenic acid ethyl ester, xanthophylls (flavoxanthin, lutein, cryptoxanthin, rubixanthin, violaxanthin and rodoxanthin), fast carmine (carmic acid, cochineal), azorubine, Cochineal Red A (Ponceau 4 R), beetroot red, betanin, anthocyans, amaranth, Patent Blue V, Indigotine I (indigo carmine), chlorophylls, copper compounds of the chlorophylls, Brilliant Acid Green BS, lissamine green), Brilliant Black BN, carbo medicinalis vegetabilis, titanium dioxide, iron oxides and hydroxides, calcium carbonate, aluminium, silver, gold, Rubine Pigment BK (Lithol Rubine BK), Methyl Violet B, Victoria Blue R, Victoria Blue B, Acilan Brilliant Blue FFR (Brilliant Wool Blue FFR), Naphthol Green B, Acilan Fast Green 10 G (Alkali Fast Green 10 G), Ceres Yellow GRN, Sudan Blue II, ultramarine, phthalocyanine blue, phthalocyanine green and Fast Acid Violet R. Further naturally obtained extracts (e.g. paprika extract, black carrot extract, red cabbage extract) can be used for colouring purposes.

The following examples are intended to illustrate the present invention without limiting it.

### Examples

Unless stated otherwise, all the data relate to the weight.

Mixtures E-1 to E-6 according to the invention (see Table 1) were employed in the formulation examples.

### In vitro test for reduction of bad breath

The test is based on the work of Goldberg and Rosenberg (Production of Oral Malodor in an in vitro System, S. Goldberg and M. Rosenberg, pp. 143 - 150, in: Bad Breath - A Multidisciplinary Approach, eds: D. van Steenberghe, M. Rosenberg, Leuven University Press, 1996) and was adapted for better reproducibility.

A sterile liquid medium inoculated with fresh morning saliva is incubated at 37° C for some days and then smelled by a panel of testers.

An intensive, typical bad breath odour had formed. Non-inoculated controls have only a weak smell of the medium.

Triclosan® (5-chloro-2-(2,4-dichlorophenoxy)phenol) was added to inoculated samples in a concentration of 0.05 % as a control for the tests. After the incubation, the inoculated and Triclosan®-treated samples had the same weak smell of the medium as the non-inoculated samples.

By addition of in each case 0.01 % of the mixtures E-1 to E-6 according to the invention (see Table 1) to inoculated samples, no smell - analogously to Triclosan® - was to be found after the incubation time. This value also corresponds to the minimum active concentration. At significantly lower concentrations than 0.01 %, a changed intrinsic smell or a mixed smell was perceptible in some cases.

If 0.05 % of a mixture according to the invention is added to the inoculated medium only one hour before the sensory evaluation, an unpleasant mixed smell of bad breath and a weak note of the mixture according to the invention is to be observed. This observation demonstrates that the action of a mixture according to the invention against bad breath is not an odour-masking / -covering action.

In further studies, in a concentration of 1,000 ppm the mixtures E-1 to E-6 according to the invention showed no significant reduction in respect of the total number of germs in the oral saliva samples investigated.

On the other hand, a bacteriostatic or bactericidal action was detected against the germ Fusobacterium nucleatum, which causes bad breath, at a concentration of 250-500 ppm.

### Determination of the minimum inhibitory concentration

The minimum inhibitory concentration (MIC) of mixture E-4 according to the invention (see Table 1) was determined in the series dilution test against various bacteria. The result is shown in the following table:

| Organism | MIC [ppm] | Type |
|---|---|---|
| Escherichia coli | > 1,000 | no action up to 1,000 ppm |
| Pseudomonas aeruginosa | > 1,000 | no action up to 1,000 ppm |
| Staphylococcus aureus | 125 | |
| Streptococcus mutans | 125 | bactericidal |
| Fusobacterium nucleatum | 250 | bactericidal |
| Prevotella intermedia | 64 | bactericidal |
| Porphyromonas gingivalis | 125 | |

A bactericidal action was thus detected against the germs Fusobacterium nucleatum and Prevotella intermedia, which cause bad breath, at a concentration of 500 ppm of mixture E-4 according to the invention. An activity against Porphyromonas gingivalis was furthermore observed, and an MIC of 125 ppm was determined. In addition, mixture E-4 according to the invention shows a bactericidal action against Streptococcus mutans, which plays a role in plaque and plaque formation, as in the development of caries.

In a further test in a concentration of 1,000 ppm, mixture E-4 according to the invention showed no significant reduction in respect of the total number of germs in the oral saliva samples investigated.

### In vivo test for reduction of bad breath

An opaque toothpaste formulation, called test toothpaste in the following, of the following composition was prepared:

| | l(%) |
|---|---|
| Na carboxymethylcellulose | 0.90 |
| Sorbitol 70 %, in water | 45.00 |
| Na saccharinate | 0.20 |
| PEG 1500 | 5.00 |
| Titanium dioxide | 0.50 |
| Methylparaben, Na salt (Solbrol M Na salt) | 0.15 |
| Trisodium phosphate | 0.10 |
| Sodium monofluorophosphate | 1.12 |
| Abrasive silica (Sident 9) | 10.00 |
| Thickening silica (Sident 22 S) | 8.00 |
| Sodium lauryl sulfate (SDS) | 1.50 |
| Dist. water | to 100.00 |

Three groups each of 20 volunteers with normal bad breath cleaned their teeth once in the context of the investigation described here (cleaning time: 2 minutes). The exhaled air of each volunteer was evaluated by three sensorially trained testers in each case on a scale from 0 (no bad breath) to 5 (very severe bad breath) immediately before and after the use and after 30 and 60 minutes.

The volunteers of group (I) cleaned their teeth with a test toothpaste into which 0.3 wt.%, based on the total weight of the test toothpaste, of mixture E-4 according to the invention had been incorporated.

The volunteers of group (II) cleaned their teeth with a test toothpaste into which 0.2 wt.% of mixture E-4 according to the invention and 0.2 wt.% of aroma A (composition below), in each case based on the total weight of the test toothpaste, had been incorporated.

The volunteers of group (III) cleaned their teeth with an antibacterially active, commercially available toothpaste which contained 0.3 wt.% Triclosan® and a fresh mint aroma.

Aroma A had the following composition:

30 wt.% I-menthol, 30 wt.% peppermint oil Mentha piperita, 21.5 wt.% peppermint oil Mentha arvensis, 9 wt.% anethole, 0.5 wt.% anisaldehyde, 2 wt.% eucalyptol, 1 wt.% Eucalyptus globulus oil, 3 wt.% menthone, 1 wt.% spearmint oil, 1 wt.% basil oil, 0.5 wt.% menthyl acetate, 0.05 wt.% menthyl lactate, 0.1 wt.% menthyl-3-carboxylic acid N-ethylamide (WS-3), 0.05 wt.% 2-hydroxyethyl menthyl carbonate (Frescolat MGC, Symrise), 0.05 wt.% 2-hydroxypropyl menthyl carbonate (Frescolat MPC, Symrise), 0.1 wt.% pinene, 0.1 wt.% propylene glycol, 0.05 wt.% limonene.

After a single use, the following sensorial results were obtained (scale: intensity of the bad breath before use = 100 %):

| | immediately after use | after 30 minutes | after 60 minutes |
|---|---|---|---|
| Group (I) | 10% | 43% | 62% |
| Group (II) | 22% | 56% | 76% |
| Group (III) (comparison) | 42% | 72% | 81% |

### Halimeter® measurement (VSC oral values)

Volatile sulfur compounds (VSC) in exhaled air play a very important role in connection with bad breath. Compounds such as hydrogen sulfide, dimethyl sulfide, methylmercaptan and other sulfur compounds cause the nauseous odour.

A quantitative determination of the VSCs of the exhaled air of volunteers was carried out by measurements with the Halimeter® apparatus from Interscan Corporation (Chatsworth, Canada). The measurements were in each case performed in the oral cavity and over the tongue. The average of the values was then taken.

A group of 20 volunteers (group 1) with severe bad breath (oral halitosis) cleaned their teeth (cleaning time: 3 minutes) twice daily over a period of 7 days with a test toothpaste into which 0.3 wt.%, based on the total weight of the test toothpaste, of mixture E-4 according to the invention had been incorporated.

A second group of 20 volunteers (group 2) with severe bad breath (oral halitosis) cleaned their teeth (cleaning time: 3 minutes) twice daily over a period of 7 days with a test toothpaste into which 0.2 wt.% of mixture E-4 according to the invention and 0.2 wt.% of aroma A (for the composition, see above), in each case based on the total weight of the test toothpaste, had been incorporated.

A third group (group 3) of 20 volunteers with severe bad breath (oral halitosis) cleaned their teeth (cleaning time: 3 minutes) twice daily over a period of 7 days with an antibacterially active, commercially available test toothpaste which contained 0.3 wt.% Triclosan® and a fresh mint aroma.

Before the start of the test phase and after the end of the one-week treatment, a quantitative determination of the volatile sulfur compounds was carried out on all the volunteers. The measurement after the one-week treatment was performed about 10 hours after the last cleaning of the teeth, on the following morning, that is to say after bad breath had been able to develop again overnight.

While no reduction in the VSCs was found for the Tridosan®-containing commercially available toothpaste (groups 3), and even rather a slight increase, in the volunteers of group I a significant reduction (p < 0.05) of the VSCs in the range of 21 - 29 % (before use: 100 %) was found. In the volunteers of group 2, a reduction of the VSCs in the range of 12 - 19 % was found.

### Formulation examples

1. Gel dental cream with an activity against bad breath

| | I (%) | II (%) | III (%) |
|---|---|---|---|
| Na carboxymethylcellulose | 0.40 | 0.40 | 0.40 |
| Sorbitol 70 %, in water | 72.00 | 72.00 | 72.00 |
| Polyethylene glycol (PEG) 1500 | 3.00 | 3.00 | 3.00 |
| Na saccharinate | 0.07 | 0.07 | 0.07 |
| Na fluoride | 0.24 | 0.24 | 0.24 |
| p-Hydroxybenzoic acid (PHB) ethyl ester | 0.15 | 0.15 | 0.15 |
| Aroma A (see above) | 0.10 | 0.80 | 0.75 |
| Mixture E-4 | 0.20 | 0.40 | 0.75 |
| Abrasive silica | 11.00 | 11.00 | 11.00 |
| Thickening silica | 6.00 | 6.00 | 6.00 |
| Sodium dodecyl sulfate (SDS) | 1.40 | 1.40 | 1.40 |
| Dist. water | to 100.00 | to 100.00 | to 100.00 |

2. Dental cream against plaque with an activity against bad breath

| | I (%) | II (%) | III (%) |
|---|---|---|---|
| Na carboxymethylcellulose | 1.00 | 1.00 | 1.00 |
| Glycerol | 12.50 | 12.50 | 12.50 |
| Sorbitol 70 %, in water | 29.00 | 29.00 | 29.00 |
| Na saccharinate | 0.20 | 0.20 | 0.20 |
| Na fluoride | 0.22 | 0.22 | 0.22 |
| Azacycloheptane-2,2-diphospho acid, di-sodium salt | 1.00 | 1.00 | 1.00 |
| Bromochlorophene | 0.10 | 0.10 | 0.10 |
| Peppermint aroma | 0.05 | 1.10 | 0.35 |
| Mixture E-1 | 0.05 | 0.30 | 0.70 |
| Abrasive silica | 15.00 | 15.00 | 15.00 |
| Thickening silica | 5.00 | 5.00 | 5.00 |
| Sodium dodecyl sulfate (SDS) | 1.50 | 1.50 | 1.50 |
| Dist. water | to 100.00 | to 100.00 | to 100.00 |

3. Dental cream against plaque with an activity against bad breath
Base: silica, alkali metal diphosphate

| | I (%) | II (%) | II (%) |
|---|---|---|---|
| Carrageenan | 0.90 | 0.90 | 0.90 |
| Glycerol | 15.00 | 15.00 | 15.00 |
| Sorbitol 70 %, in water | 25.00 | 25.00 | 25.00 |
| PEG 1000 | 3.00 | 3.00 | 3.00 |
| Na fluoride | 0.24 | 0.24 | 0.24 |
| Tetrapotassium diphosphate | 4.50 | 4.50 | 4.50 |
| Tetrasodium diphosphate | 1.50 | 1.50 | 1.50 |
| Na saccharinate | 0.40 | 0.40 | 0.40 |
| Precipitated silica | 20.00 | 20.00 | 20.00 |
| Titanium dioxide | 1.00 | 1.00 | 1.00 |
| PHB methyl ester | 0.10 | 0.10 | 0.10 |
| Menthol/eucalyptol aroma | 1.10 | 0.80 | 0.20 |
| Mixture E-3 | 0.10 | 0.40 | 1.00 |
| Sodium dodecyl sulfate | 1.30 | 1.30 | 1.30 |
| Dist. water | to 100.00 | to 100.00 | to 100.00 |

4. Dental cream against sensitive teeth with an activity against bad breath

| | I (%) | II (%) | III (%) |
|---|---|---|---|
| Na carboxymethylcellulose | 0.70 | 0.70 | 0.70 |
| Xanthan gum | 0.50 | 0.50 | 0.50 |
| Glycerol | 15.00 | 15.00 | 15.00 |
| Sorbitol 70 %, in water | 12.00 | 12.00 | 12.00 |
| K nitrate | 5.00 | 5.00 | 5.00 |
| Na monofluorophosphate | 0.80 | 0.80 | 0.80 |
| PHB methyl ester | 0.15 | 0.15 | 0.15 |
| PHB propyl ester | 0.05 | 0.05 | 0.05 |
| Na saccharinate | 0.20 | 0.20 | 0.20 |
| Menthol/anethole aroma | 0.25 | 0.75 | 0.25 |
| Mixture E-2 | 0.05 | 0.25 | 0.75 |
| Ca carbonate | 35.00 | 35.00 | 35.00 |
| Silicon dioxide | 1.00 | 1.00 | 1.00 |
| Sodium dodecyl sulfate (SDS) | 1.50 | 1.50 | 1.50 |
| Dist. water | to 100.00 | to 100.00 | to 100.00 |

5. Dental cream against sensitive teeth with an activity against bad breath

| | I (%) | II (%) | III (%) |
|---|---|---|---|
| Hydroxyethylcellulose | 1.40 | 1.40 | 1.40 |
| Guar gum | 0.60 | 0.60 | 0.60 |
| Glycerol | 18.00 | 18.00 | 18.00 |
| Sorbitol 70 %, in water | 12.00 | 12.00 | 12.00 |
| Na saccharinate | 0.35 | 0.35 | 0.35 |
| Dyestuff | 0.01 | 0.01 | 0.01 |
| PHB methyl ester | 0.15 | 0.15 | 0.15 |
| PHB propyl ester | 0.04 | 0.04 | 0.04 |
| Sr chloride | 10.50 | 10.50 | 10.50 |
| Peppermint/aniseed aroma | 0.35 | 1.20 | 0.60 |
| Mixture E-6 | 0.05 | 0.50 | 0.90 |
| Precipitated silica | 15.00 | 15.00 | 15.00 |
| Silicon dioxide | 1.60 | 1.60 | 1.60 |
| Sodium dodecyl sulfate | 1.30 | 1.30 | 1.30 |
| Dist. water | to 100.00 | to 100.00 | to 100.00 |

6. Ready-to-use mouth wash with fluoride and an activity against bad breath

| | I (%) | II (%) | III (%) |
|---|---|---|---|
| Ethanol | 7.00 | 7.00 | 7.00 |
| Glycerol | 12.00 | 12.00 | 12.00 |
| Na fluoride | 0.05 | 0.05 | 0.05 |
| Pluronic F-127^{®} (BASF, surface-active substance) | 1.40 | 1.40 | 1.40 |
| Na phosphate buffer pH 7.0 | 1.10 | 1.10 | 1.10 |
| Sorbic acid | 0.20 | 0.20 | 0.20 |
| Na saccharinate | 0.10 | 0.10 | 0.10 |
| Menthol/peppermint aroma | 0.08 | 0.20 | 0.15 |
| Mixture E-4 | 0.02 | 0.20 | 0.10 |
| Dyestuff | 0.01 | 0.01 | 0.01 |
| Dist. water | to 100.00 | to 100.00 | to 100.00 |

7. Mouth wash concentrate with an activity against bad breath

| | I(%) | II (%) | III (%) |
|---|---|---|---|
| Ethanol, 95 % strength | 80.00 | 80.00 | 80.00 |
| Na cyclamate | 0.15 | 0.15 | 0.15 |
| Menthol/aniseed/ eucalyptol aroma | 1.50 | 2.00 | 2.00 |
| Dyestuff | 0.01 | 0.01 | 0.01 |
| Mixture E-3 | 1.50 | 2.50 | 3.00 |
| Dist. water | to 100.00 | to 100.00 | to 100.00 |

8. Chewing gum against bad breath

| | I (%) | II (%) | III (%) |
|---|---|---|---|
| Chewing gum base | 21.00 | 21.00 | 21.00 |
| Glucose syrup | 16.50 | 16.50 | 16.50 |
| Glycerol | 0.50 | 0.50 | 0.50 |
| Powdered sugar | 60.45 | 60.40 | 60.30 |
| Menthol/spearmint aroma | 1.20 | 1.00 | 0.70 |
| Mixture E-6 | 0.35 | 0.60 | 1.00 |

9. Sugar-free chewing gum against bad breath

| | I (%) | I (%) | III (%) |
|---|---|---|---|
| Chewing gum base | 30.00 | 30.00 | 30.00 |
| Sorbitol, powder | 38.45 | 38.40 | 38.30 |
| Palatinite | 9.50 | 9.50 | 9.50 |
| Xylitol | 2.00 | 2.00 | 2.00 |
| Mannitol | 3.00 | 3.00 | 3.00 |
| Aspartame | 0.10 | 0.10 | 0.10 |
| Acesulfame K | 0.10 | 0.10 | 0.10 |
| Emulgum / emulsifier | 0.30 | 0.30 | 0.30 |
| Sorbitol 70 %, in water | 14.00 | 14.00 | 14.00 |
| Glycerol | 1.00 | 1.00 | 1.00 |
| Menthol/aniseed/ cinna-mon aroma | 1.20 | 0.80 | 0.60 |
| Mixture E-4 | 0.35 | 0.80 | 1.10 |

10. Gelatine capsules against bad breath for direct consumption

| | I(%) | II(%) | III (%) |
|---|---|---|---|
| Gelatine shell: | | | |
| Glycerol | 2.014 | 2.014 | 2.014 |
| Gelatine 240 Bloom | 7.91 | 7.91 | 7.91 |
| Sucralose | 0.065 | 0.065 | 0.065 |
| Allura Red | 0.006 | 0.006 | 0.006 |
| Brilliant Blue | 0.005 | 0.005 | 0.005 |
| | | | |

| Core composition: | | | |
|---|---|---|---|
| Plant oil triglyceride (coconut oil fraction) | 80.0 | 70.0 | 66.0 |
| Aroma B | 7.0 | 12.0 | 12.0 |
| Mixture E-4 | 3.0 | 8.0 | 12.0 |

Aroma B here had the following composition (data in each case in wt.%):
0.1 % neotame powder, 0.05 % aspartame, 29.3 % peppermint oil arvensis, 29.3 % peppermint piperita oil Willamette, 2.97 % sucralose, 2.28 % triacetin, 5.4 % diethyl tartrate, 12.1 % peppermint oil yakima, 0.7 % ethanol, 3.36 % 2-hydroxyethyl menthyl carbonate, 3.0 % 2-hydroxypropyl menthyl carbonate, 0.27 % vanillin, 5.5% D-limonene, 5.67% L-menthyl acetate.

The gelatine capsule, which is suitable for direct consumption, had a diameter of 5 mm, and the weight ratio of core material to shell material was 90 : 10. The capsules opened in the mouth within less than 10 seconds and dissolved completely within less than 50 seconds.

## Claims

1. Mixture comprising (a) eugenol acetate and isoeugenol methyl ether or (B) eugenol acetate and beta-homocyclocitral or (c) eugenol acetate, isoeugenol methyl ether and beta-homocyclocitral.

2. Mixture according to claim 1, comprising
(a) eugenol acetate and isoeugenol methyl ether, wherein the weight ratio of eugenol acetate to isoeugenol methyl ether is in the range of from 10 : 1 to 1 : 10 or
(b) eugenol acetate and beta-homocyclocitral, wherein the weight ratio of eugenol acetate to beta-homocyclocitral is in the range of from 100 : 1 to 1 5 or
(c) eugenol acetate, isoeugenol methyl ether and beta-homocyclocitral, wherein the weight ratio of eugenol acetate to isoeugenol methyl ether is in the range of from 10 : 1 to 1 : 10 and the weight ratio of eugenol acetate to beta-homocyclocitral is in the range of from 100 : 1 to 1 : 5.

3. Mixture according to claim 1 or 2, therein the total content of the constituents eugenol acetate, isoeugenol methyl ether and beta-homocyclocitral in the mixture is in the range of from 10 to 100 wt.%, based on the total weight of the mixture.

4. Mixture according to one of claims 1-3, comprising; eugenol acetate, isoeugenol methyl ether, at least one further substance chosen from the group consisting of farnesol, myristicin, apiole, parsley oil and thymol, and optionally beta-homocyclocitral.

5. Mixture according to one of claims 1-4, furthermore comprising menthol and optionally one or more substances having an aniseed note and/or eucalyptol and/or one or more herbal components, in each case in a sensorially active amount,.

6. Use of a mixture according to one of claims 1-5 as an agent for inhibiting and/or preventing the growth of and/or for killing microorganisms which cause bad breath.

7. Use according to claim 6, wherein the microorganisms which cause bad breath are chosen from the group consisting of: Eubacterium, Fusobacterium, Haemophilus, Neisseria, Porphyromonas, Prevotella, Treponema and Veillonella species.

8. Oral hygiene product containing a mixture according to one of claims 1-5 in an amount of at least 0,01 wt.%, based on the total weight of the oral hygiene product.

9. Method of non-therapeutically inhibiting and/or preventing the growth of and/or killing microorganisms which cause bad breath with the following step:
- bringing microorganisms which cause bad breath into contact with a mixture according to one of claims 1-5 in an amount which is antimicrobially active against these microorganisms.

10. Method of non-therapeutically combating bad breath with the following step
- introduction of a mixture according to one of claims 1-5 in an amount which is antimicrobially active against microorganisms which cause bad breath into the oral cavity and/or the pharyngeal cavity.

## Patentansprüche

1. Mischung, umfassend (a) Eugenolacetat und Isoeugenolmethylether oder (b) Eugenolacetat und beta-Homocyclocitral oder (c) Eugenolacetat, Isoeugenolmethylether und beta-Homocyclocitral.

2. Mischung nach Anspruch 1, umfassend
(a) Eugenolacetat und Isoeugenolmethylether, wobei das Gewichtsverhältnis von Eugenolacetat zu Isoeugenolmethylether im Bereich von 10:1 bis 1:10 liegt, oder
(b) Eugenolacetat und beta-Homocyclocitral, wobei das Gewichtsverhältnis von Eugenolacetat zu beta-Homocyclocitral im Bereich von 100:1 bis 1:5 liegt, oder
(c) Eugenolacetat, Isoeugenolmethylether und beta-Homocyclocitral, wobei das Gewichtsverhältnis von Eugenolacetat zu Isoeugenolmethylether im Bereich von 10:1 bis 1:10 liegt und das Gewichtsverhältnis von Eugenolacetat zu beta-Homocyclocitral im Bereich von 100:1 bis 1:5 liegt.

3. Mischung nach Anspruch 1 oder 2, wobei der Gesamtgehalt der Bestandteile Eugenolacetat, Isoeugenolmethylether und beta-Homocyclocitral in der Mischung im Bereich von 10 bis 100 Gew.%, bezogen auf das Gesamtgewicht der Mischung, liegt.

4. Mischung nach einem der Ansprüche 1 bis 3, umfassend: Eugenolacetat, Isoeugenolmethylether, mindestens eine weitere Substanz, ausgewählt aus Farnesol, Myristicin, Apiol, Petersilienöl und Thymol, und gegebenenfalls beta-Homocyclocitral.

5. Mischung nach einem der Ansprüche 1 bis 4, weiterhin umfassend Menthol und gegebenenfalls eine oder mehrere Substanzen mit Anisnote und/oder Eucalyptol und/oder eine oder mehrere Kräuterkomponenten, jeweils in einer sensorisch wirksamen Menge.

6. Verwendung einer Mischung nach einem der Ansprüche 1 bis 5 als Mittel zur Hemmung und/oder Verhinderung des Wachstums und/oder zur Abtötung von Mikroorganismen, welche Mundgeruch verursachen.

7. Verwendung nach Anspruch 6, wobei die Mikroorganismen, die Mundgeruch verursachen, ausgewählt sind aus: Eubacterium-, Fusobacterium-, Haemophilus-, Neisseria-, Porphyromonas-, Prevotella-, Treponema- und Veillonella-Arten.

8. Mundhygieneprodukt, enthaltend eine Mischung nach einem der Ansprüche 1 bis 5 in einer Menge von mindestens 0,01 Gew.-%, bezogen auf das Gesamtgewicht des Mundhygieneproduktes.

9. Verfahren zur nichttherapeutischen Hemmung und/oder Verhinderung des Wachstums und/oder Abtötung von Mikroorganismen, die Mundgeruch verursachen, mit dem folgenden Schritt:
- Inkontaktbringen der Mikroorganismen, die Mundgeruch verursachen, mit einer Mischung nach einem der Ansprüche 1 bis 5 in einer Menge, die antimikrobiell wirksam gegen diese Mikroorganismen ist.

10. Verfahren zur nichttherapeutischen Bekämpfung von Mundgeruch mit dem folgenden Schritt:
- Einbringen einer Mischung nach einem der Ansprüche 1 bis 5 in einer Menge, die antimikrobiell wirksam gegen Mikroorganismen ist, die Mundgeruch verursachen, in die Mundhöhle und/oder die Rachenhöhle.

## Revendications

1. Mélange comprenant (a) de l'acétate d'eugénol et du méthyléther d'isoeugénol ou (b) de l'acétate d'eugénol et du bêta-homocyclocitral ou (c) de l'acétate d'eugénol, du méthyléther d'isoeugénol et du bêta-homocyclocitral.

2. Mélange selon la revendication 1, comprenant:
(a) de l'acétate d'eugénol et du méthyléther d'isoeugénol, où le rapport en poids de l'acétate d'eugénol sur le méthyléther d'isoeugénol est dans l'intervalle de 10:1 à 1:10 ou
(b) de l'acétate d'eugénol et du bèta-homocyclocitral, où le rapport en poids de l'acétate d'eugénol sur le bêta-homocyclocitral est dans l'intervalle de 100:1 à 1:5 ou
(c) de l'acétate d'eugénol, du méthyléther d'isoeugénol et du bêta-homocyclocitral, otl le rapport en poids de l'acétate d'eugénol sur le méthyléther d'isoeugénol est dans l'intervalle de 10:1 à 1:10 et le rapport en poids de l'acétate d'eugénol sur le bêta-homocyclocitral est dans l'intervalle de 100:1 à 1:5.

3. Mélange selon la revendication 1 ou 2, dans lequel la teneur totale des constituants acétate d'eugénol, méthyléther d'isoeugénol et bêta-homocyclocitral dans le mélange est dans l'intervalle de 10 à 100% en poids, sur la base du poids total du mélange.

4. Mélange selon l'une des revendications 1-3, comprenant: de l'acétate d'eugénol, du méthyléther d'isoeugénol, au moins une substance supplémentaire choisie dans le groupe constitué de farnésol, de myristicine, d'apiole, d'huile de persil et de thymol, et éventuellement du bêta-homocyclocitral.

5. Mélange selon l'une des revendications 1-4, comprenant en outre du menthol et éventuellement une ou plusieurs substances possédant une note anisée et/ou de l'eucalyptol et/ou un ou plusieurs composants d'herbes, dans chaque cas dans une quantité sensoriellement active.

6. Utilisation d'un mélange selon l'une des revendications 1-5 en tant qu'agent pour inhiber et/ou prévenir la croissance de et/ou pour tuer des microorganismes qui provoquent une mauvaise haleine.

7. Utilisation selon la revendication 6, dans laquelle les microorganismes qui provoquent une mauvaise haleine sont choisis dans le groupe constitué: des espèces Eubacterium, Fusobacterium, Haemophilus, Neisseria, Porphyromonas, Prevotella, Treponema et Veillonella.

8. Produit d'hygiène buccale contenant un mélange selon l'une des revendication 1-5 dans une quantité d'au moins 0,01% en poids, sur la base du poids total du produit d'hygiène buccale.

9. Méthode pour inhiber et/ou prévenir de manière non thérapeutique la croissance de et/ou tuer des microorganismes qui provoquent une mauvaise haleine avec l'étape suivante:
- de mise en contact de microorganismes qui provoquent une mauvaise haleine avec un mélange selon l'une des revendications 1-5 dans une quantité qui est active du point de vue antimicrobien contre ces microorganismes.

10. Méthode pour combattre de manière non thérapeutique une mauvaise haleine avec l'étape suivante:
- d'introduction d'un mélange selon l'une des revendications 1-5 dans une quantité qui est active du point de vue antimicrobien contre des microorganismes qui provoquent une mauvaise haleine dans la cavité buccale et/ou la cavité pharyngée.
